# EUROPÄISCHE PATENTSCHRIFT

(11) **EP 0 671 379 B1**
(45) Veröffentlichungstag und Bekanntmachung des Hinweises auf die Patenterteilung: **13.08.1997**
(21) Anmeldenummer: 95102942.0
(22) Anmeldetag: 02.03.1995
(51) Int. Cl.: C07C 45/68, C07C 67/343, C07C 231/12, C07C 253/12, C07C 315/04, C07B 37/02

(54) **Verfahren zur Methylierung organischer Verbindungen**
Process for the methylation of organic compounds
Procédé de méthylation de composés organiques

(30) Priorität: 07.03.1994 DE 4407495
(43) Veröffentlichungstag der Anmeldung: 13.09.1995
(73) Patentinhaber: BASF AKTIENGESELLSCHAFT, 67056 Ludwigshafen (DE)
(72) Erfinder: Fischer, Rolf, Dr., D-69121 Heidelberg (DE)

(56) Entgegenhaltungen:
- EP-A- 0 240 863
- EP-A- 0 525 506
- EP-A- 0 602 515

## Beschreibung

Die vorliegende Erfindung betrifft ein neues Verfahren zur Herstellung von Methylverbindungen der allgemeinen Formel I 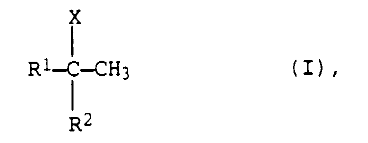 in der die Substituenten folgende Bedeutung haben:
- R¹, R²: Wasserstoff, unabhängig voneinander oder gemeinsam organischer Rest mit 1 bis 40 Kohlenstoffatomen,
- R²: 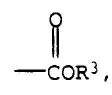 ―CN,
- X: 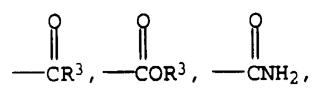 -CN, - SO₂R⁴,
wobei R³ für Wasserstoff oder C₁-C₆-Alkyl steht, R⁴ für C₂-C₆-Alkyl steht oder mit R¹ ein C₃-C₅-Alkylen- oder Alkenylenbrückenglied bildet.

Carbonsäureester und Nitrile können in α-Stellung zur funktionellen Gruppe alkyliert werden. Hierzu müssen diese Verbindungen zunächst mit starken Basen wie Alkalialkoholaten, Alkalimetallamiden, Alkalihydriden oder lithiumorganischen Verbindungen in die entsprechenden Anionen überführt werden. Diese lassen sich anschließend durch Umsetzung mit Alkylierungsmitteln, z.B. Alkylhalogeniden, in die gewünschten α-alkylierten Verbindungen überführen (Houben-Weyl, Methoden der organischen Chemie, vierte Auflage, Band E 5, 364-373 und 1495-1505). Nachteilig an diesen Methoden ist, daß sie mit Salzanfall verbunden sind.

Die deutsche Patentanmeldung P 42 42 451.8 betrifft die Methylierung von Butyrolacton mit Dimethylcarbonat in Gegenwart einer stickstoffhaltigen Base. Die EP-A 525 506 lehrt die Methylierung von Arylacetonitrilen, Arylessigsäuren und deren Estern mit Dimethylcarbonat in Gegenwart von Alkalimetallcarbonaten und Bicarbonaten in der Flüssigphase. Ein kontinuierliches Verfahren zur Alkylierung CH-acider Verbindungen mit Alkylcarbonaten in der Gas/Flüssig-Phase unter Phasentransferkatalyse wird in der EP-A-240 863 beschrieben.

Der vorliegenden Anmeldung lag die Aufgabe zugrunde, ein breit anwendbares, ohne die Verwendung großer Mengen an Mineralsalzen bzw. ohne Mineralsalzanfall durchführbares Methylierungsverfahren für CH-acide Verbindungen zu entwickeln.

Demgemäß wurde das oben definierte Verfahren gefunden, das dadurch gekennzeichnet ist, daß man
a) eine Verbindung der allgemeinen Formel II 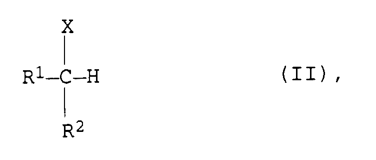 oder
b) für den Fall, daß R² in Verbindung I Wasserstoff bedeutet, eine Verbindung der allgemeinen Formel III 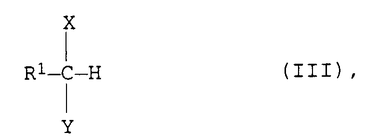 in der Y für 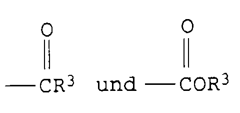 steht,
mit Dimethylcarbonat in Gegenwart einer stickstoffhaltigen Base umsetzt.

Die erfindungsgemäße Umsetzung läßt sich für den Fall der Methylierung von Valeronitril (Verfahrensvariante (a)) zu 2-Methylvaleronitril durch die Reaktionsgleichung 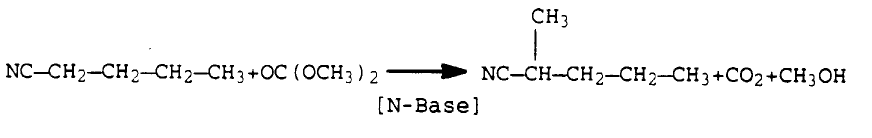 und für den Fall der Methylierung von (n-Butyl)-cyanessigsäuremethylester zu 2-Methylcapronitril (Verfahrensvariante (b)) durch die Reaktionsgleichung 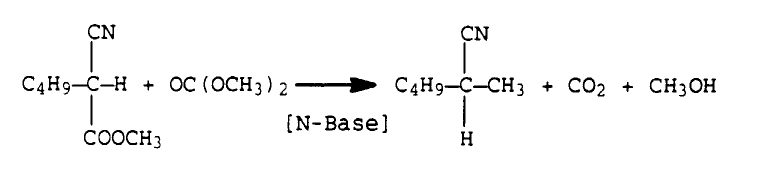 veranschaulichen.

Bei den Ausgangsverbindungen II und III handelt es sich um CH-acide Verbindungen. Diese tragen eine elektronenziehende Gruppe X oder Y entweder in Form einer Carbonylgruppe, z.B. einer Formylgruppe, einer Acylgruppe wie Acetyl, einer Carboxygruppe, einer Carboxyestergruppe wie Methoxycarbonyl und Ethoxycarbonyl, einer Carbonylgruppe oder in Form einer Cyanogruppe oder einer Alkylsulfonylgruppe. Die Substituenten R¹ und R² in Verbindung II bzw. R¹ in Verbindung III haben keinen erkennbaren Einfluß auf die Reaktion, solange es sich dabei um Reste handelt, die unter onsbedingung inert sind.

R¹ und R² können deshalb unabhängig voneinander für Wasserstoff sowie für organische Reste mit 1 bis 40 Kohlenstoffatome stehen. Im einzelnen kann es sich dabei um folgende Reste handeln:
- Cycloalkylgruppen, vorzugsweise mit 3 bis 7 Kohlenstoffatomen, wie Cyclopropyl, Cyclohexyl,
- Aralkylgruppen, vorzugsweise C₇-C₁₂-Aralkylgruppen wie Benzyl,
- Aryl, vorzugsweise C₆-C₁₀-Aryl, wie Phenyl,
- Heteroaryl wie Furyl, Thienyl, Pyrryl, Pyridyl,
- aliphatisch-cycloaliphatische Gruppen wie 2-Cyclohexylpropylen,
- Alkenylgruppen, vorzugsweise C₃-C₈-Alkenyl wie Propenyl, Butenyl.

Diese aliphatischen wie aromatischen Reste können einen oder mehrere inerte Substituenten tragen wie Halogen, z.B. Fluor, Chlor und Brom, Alkoxy, vorzugsweise C₁-C₆-Alkoxy wie Methoxy, Ethoxy und Propoxy, aber auch Alkoxycarbonyl wie in (CH₂)ₙCOOR⁵, wobei n eine ganze Zahl von 1 bis 6 ist und R⁵ für eine C₁-C₆-Alkylgruppe steht, und Cyanogruppen wie in (CH₂)ₙCN.

Weiterhin können R¹ und R² gemeinsam eine C₂-C₁₀-Alkylenkette bilden, die gegebenenfalls ein Sauerstoffatom, Schwefelatom, eine NR³-Gruppe oder -SO₂- enthalten kann. Schließlich kann R¹ in dem Fall, daß X für -SO₂- steht, mit x eine C₃-C₅-Alkylen- und Alkenylenbrücke bilden.

Die als Ausgangsverbindungen verwendeten Nitrile, Carbonsäureester, 1,3-Diketone, Cyancarbonsäureester, Formylcarbonsaureester, Carbonsäureamide und Sulfone der allgemeinen Formel II und III sind allgemein zugängliche Verbindungen, die z.B. nach Houben-Weyl, Methoden der Organischen Chemie, Thieme Verlag herstellbar sind.

Im einzelnen kommen folgende Verbindungsklassen und Verbindungen als Ausgangsverbindungen in Betracht:
- Nitrile der allgemeinen Formel II, z.B. Acetonitril, Propionitril, n-Butyronitril, n-Valeronitril, 3-Phenylpropionitril, 3-Methylbutyronitril, 4-Cyclohexylvaleronitril, Benzylnitril, p-Methylbenzylnitril, p-Methoxybenzylnitril, Adipodinitril;
- Carbonsäureester und 1,3-Diketoverbindungen der allgemeinen Formeln II und III, z.B. Essigsäuremethylester, Propionsäure-n-butylester, Valeriansäuremethylester, Phenylessigsäuremethylester, Cyclohexylessigsäuremethylester, Bernsteinsäurepropylester, Palmitinsäuremethylester, 3- und 4-Pentensäuremethylester, Malonsäuredimethylester, 2-Methylmalonsäuredimethylester, 2-Phenylmalonsäuredimethylester, 2-(2-Methoxycarbonylethyl)-malonsäuredimethylester, 2-Benzylmalonsäuredimethylester, Adipinsäuredimethylester, Bernsteinsäuredimethylester, Glutarsäuredimethylester, Acetessigester, 2-(n-Butyl)-acetessigester, Acetylaceton, 3-Ethylacetylaceton, 2-Formylpropionsäuremethylester, 2-Formylbernsteinsäuredimethylester;
- Cyancarbonsäureester der allgemeinen Formeln II und III, z.B. 5-Cyanvaleriansäuremethylester, Cyanessigsäuremethylester, Cyanessigsäureethylester, 2-Methylcyanessigsäuremethylester, 2-n-Butylcyanessigsäuremethylester, 2 -Phenylcyanessigsäuremethylester, 2-Benzylcyanessigsäureethylester, 2-(2-Cyanoethyl)-cyanessigsäuremethylester, 2-(2-Methoxycarbonylethyl)-cyanessigsäuremethylester, 2-(1-Propenyl)-cyanessigsäuremethylester;
- Sulfone der allgemeinen Formel II, z.B. Sulfolan, Sulfolen, Di-n-Butylsulfon, Diethylsulfon.

Das erfindungsgemäße Verfahren läßt sich wie folgt durchführen:
Man kann die Verbindungen der allgemeinen Formel II bzw. III mit Dimethylcarbonat in Gegenwart von stickstoffhaltigen Basen in der Regel bei Temperaturen von 50 bis 300°C, bevorzugt 100 bis 250°C, besonders bevorzugt 150 bis 230°C und Drücken von 0,01 bis 100 bar, bevorzugt 5 bis 50 bar, besonders bevorzugt bei dem sich im jeweiligen Reaktionsgemisch einstellenden Druck umsetzen.

Im allgemeinen ist die Reaktion nach 0,5 bis 10 Stunden beendet.

Die Umsetzung kann in der Gasphase, bevorzugt aber in der Flüssigphase diskontinuierlich oder kontinuierlich durchgeführt werden, z.B. in einem Autoklaven.

Es kann zur Herstellung von luft- oder hydrolyseempfindlichen Verbindungen vorteilhaft sein, die Umsetzung in Gegenwart von unter den Reaktionsbedingungen inerten Gasen wie Stickstoff oder Argon durchzuführen.

Die Umsetzung der Verbindungen II bzw. III in der Flüssigphase kann beispielsweise so durchgeführt werden, daß man ein Gemisch aus II bzw. III und gewünschtenfalls einem Lösungsmittel in Gegenwart von Dimethylcarbonat und einer stickstoffhaltigen Base auf die gewünschte Reaktionstemperatur erhitzt. Nach beendeter Reaktion kann das Reaktionsgemisch abgekühlt und zur Gewinnung der gewünschten Verbindungen I fraktionierend destilliert werden.

Die erfindungsgemäße Umsetzung kann in Abwesenheit von Lösungsmitteln durchgeführt werden. Es kann jedoch vorteilhaft sein, in Anwesenheit von Lösungsmitteln zu arbeiten. Als Lösungsmittel können beispielsweise acyclische oder cyclische Ether wie Diethylether, Tetrahydrofuran und Dioxan, aromatische Kohlenwasserstoffe wie Benzol, Toluol und Xylol, chlorierte Kohlenwasserstoffe wie Chloroform und Methylenchlorid verwendet werden.

Die Menge an Lösungsmittel beträgt, bezogen auf die eingesetzten Verbindungen II bzw. III, 0 bis 90 Gew.-%, bevorzugt 0 bis 30 Gew.-%. Das Molverhältnis von Dimethylcarbonat zu einer Verbindung II bzw. III beträgt in der Regel 1:1 bis 10:1, bevorzugt 2:1 bis 5:1. Es ist möglich, in überschüssigem Dimethylcarbonat als Lösungsmittel zu arbeiten.

Als stickstoffhaltige Basen eignen sich Ammoniak, primäre, sekundäre und tertiäre Amine mit aliphatischen, cycloaliphatischen, heteroaromatischen und/oder araliphatischen Substituenten. Dabei können zwei aliphatische Substituenten auch zu einem Ring geschlossen sein. Weiterhin kommen Amine, die funktionelle Gruppen wie Hydroxygruppen tragen, und Polyamine in Betracht.

Im einzelnen kommen folgende Amine in Betracht:
- Ammoniak,
- Methylamin, Ethylamin, Hexylamin und Cyclohexylamin,
- Dimethylamin, Diethylamin, Dibutylamin und Dicyclohexylamin,
- Trimethylamin, Dimethylethylamin, Triethylamin, Tri-n-propylamin, Triisopropylamin, Tributylamin, Trioctylamin, Tricyclohexylamin,Trihexadecylamin, Tricyclohexylamin, Diphenylmethylamin,Dimethylbenzylamin, Dibenzylmethylamin, Tribenzylamin,
- N,N-Tetramethylhexamethylendiamin, Hexamethylendiamin, Tetramethylendiamin,
- Ethanolamin
- 4-Dimethylaminopyridin, Urotropin, Piperidin, N-Methylpiperidin, Pyrrolidin, N-Methylpyrrolidin, Hexamethylenimin, N-Ethylhexamethylenimin, N-Methylimidazol, 1,4-Diazabicyclo[2.2.2]octan (DABCO), Morpholin, Piperazin und Pyrrolidin.

Bevorzugt sind primäre, sekundäre und tertiäre Amine, die C₁- bis C₃₀-Alkyl-, C₃-C₈-Cycloalkyl- oder C₇-C₂₀-Aralkylreste tragen, sowie Amidine wie 1,5-Diazabicyclo[4.3.0]non-5-en (DBN), 1,8-Diazabicyclo-[5.4.0]undec-7-en (DBU) und Guanidin.

Besonders bevorzugt sind tertiäre Amine, insbesonders C₃- bis C₂₄-Trialkylamine.

Das Molverhältnis der Verbindungen II bzw. III zu den stickstoffhaltigen Basen beträgt in der Regel 1:1 bis 100:1, bevorzugt 3:1 bis 20:1.

Werden als Ausgangsverbindung für das erfindungsgemäße Verfahren Verbindungen der allgemeinen Formel III verwendet, ist es in einigen Fällen möglich, im Reaktionsgemisch Zwischenprodukte der Formel I stark anzureichern, in denen die Reste R² und X für -COOCH₃ und/oder -CN stehen. Dies gelingt besonders in solchen Fällen, in denen X für -CN steht. Diese Verbindungen sind teilweise aus dem Reaktionsgemisch destillativ isolierbar und können für Folgereaktionen eingesetzt werden. Eine hohe Ausbeute an solchen Zwischenprodukten läßt sich insbesondere dann erzielen, wenn die Reaktionstemperatur unter der für einen hohen Methylierungsgrad notwendigen Temperatur liegt oder wenn eine kürzere Verweilzeit als für eine vollständige Methylierung gewählt wird.

Die Verfahrensprodukte sind gesuchte Verbindungen für die Herstellung einer Vielzahl von Folgeprodukten, z.B. für die Herstellung entzündungshemmender Pharmazeutika wie Naproxen, Ibuprofen und Flurbiprofen (EP-A 525 506).

### Beispiele

Die im folgenden genannten Mengen an Ausgangsverbindungen wurden - wenn nicht anders vermerkt - 5 h bei 200°C unter Eigendruck (ca. 5 bis 80 bar) unter Rühren umgesetzt und das so erhaltene Reaktionsgemisch wurde fraktionierend destilliert oder direkt gaschromatographisch analysiert.

### I Verfahrensvariante (a))

### Beispiel 1

2-Methylvaleronitril aus Valeronitril

| | |
|---|---|
| 21 g | Valeronitril |
| 114 g | Dimethylcarbonat (DMC) |
| 9,5 g | Ethyldimethylamin |

- Ausbeute:: 10,6 g 2-Methylvaleronitril (43 %), Selektivität 59 %

### Beispiel 2

### 2-Methyladipinsäuredimethylester aus Adipinsäuredimethylester

| | |
|---|---|
| 43,5 g | Adipinsäuredimethylester |
| 115 g | DMC |
| 3,7 g | Ethyldimethylamin |

Der Reaktionsaustrag enthielt Ausgangsmaterial, 2-Methyladipinsäuredimethylester und 2,5-Dimethyladipinsäuredimethylester im Verhältnis 68:18:14 (GC-Flächenprozent).

### Beispiel 3

### 2-Methylsulfolan aus Sulfolan

| | |
|---|---|
| 45,4 g | Sulfolan |
| 90 g | DMC |
| 3 g | Ethyldimethylamin |

- Ausbeute:: 2-Methylsulfolan 33 %
Selektivität 63 %

### Beispiel 4a

### α-Methylbenzylcyanid aus Benzylcyanid

| | |
|---|---|
| 23,4 g | Benzylcyanid |
| 90 g | DMC |
| 2,9 g | Ethyldimethylamin |

- Ausbeute:: α-Methylbenzylcyanid 60 %,
α,α-Dimethylbenzylcyanid 29 %

### Beispiel 4b

### Temperatursenkung gegenüber Beispiel 4a

| | |
|---|---|
| 38,6 g | Benzylcyanid |
| 30 g | DMC |
| 4,8 g | Ethyldimethylamin |

Durchführung wie Beispiel 4a, aber bei 150°C Reaktionstemperatur.

### Analyse (GC-Flächenprozent):

| | |
|---|---|
| 46 % | 2-Methylbenzylcyanid, |
| 24 % | DMC, |
| 23 % | Benzylcyanid, |
| 7 % | nicht identifiziert, |
| <0,1 % | 2,2-Dimethylbenzylcyanid |

### Beispiel 5

### α-Methyl-di-n-butylsulfon aus Di-n-butylsulfon

| | |
|---|---|
| 35,6 g | Dibutylsulfon |
| 30 g | DMC |
| 2,9 g | Ethyldimethylamin. |

Destillation ergab bei 100 bis 114°C/0,5 mbar 31,8 g Destillat, das nach gaschromatographischer Analyse 62 % Ausgangsprodukt, 32 % α-Methyl-di-n-butylsulfon und 5 % α,α'-Dimethyl-di-n-butylsulfon enthielt.

### II Verfahrensvariante (b)

### Beispiel 6

### 2-Methylcapronitril aus 2-(n-Butyl)-Cyanessigsäuremethylester

| | |
|---|---|
| 38,8 g | 2-(n-Butyl)-Cyanessigsäuremethylester |
| 33,8 g | DMC |
| 3,6 g | Ethyldimethylamin |

- Ausbeute:: 2-Methylcapronitril 87 %,

### Beispiel 7

### 2-Methylpropionitril aus Cyanessigsäuremethylester

| | |
|---|---|
| 26 g | Cyanessigsäuremethylester |
| 117 g | DMC |
| 3,9 g | Ethyldimethylamin |

Der Reaktionsaustrag (113 g) enthielt (GC-Analyse) 48 % DMC, 10 % Methanol, 2 % Propionitril, 32 % 2-Methylpropionitril, 8 % unbekannte Verbindungen.

### Beispiel 8

### Propionsäure- und iso-Buttersäuremethylester aus Malonsäuredimethylester

| | |
|---|---|
| 3,3 g | Malonsäuredimethylester |
| 11,4 g | DMC |
| 0,38 g | Ethyldimethylamin |

Der Reaktionsaustrag enthielt Propionsäuremethylester sowie iso-Buttersäuremethylester im Verhältnis 49:51 (GC-Flächenprozent).

### Beispiel 9

### α-Methylbenzylcyanid aus 2-Phenylcyanessigsäureethylester

| | |
|---|---|
| 47,3 g | α-Methylbenzylcyanid |
| 33,8 g | DMC |
| 3,6 g | Ethyldimethylamin |

- Ausbeute:: α-Methylbenzylcyanid 32 %,
α,α-Dimethylbenzylcyanid 28 %

### Beispiel 10

### 2-Methylcapronsäuremethylester und 3-Methylheptan-2-on aus 2-(n-Butyl)-acetessigsäuremethylester

| | |
|---|---|
| 68,4 g | 2-Methylcapronsäuremethylester |
| 43,2 g | DMC |
| 5,8 g | Ethyldimethylamin |

Durch Destillation wurde ein Gemisch isoliert, das zu 27 % aus 2-Methylcapronsäuremethylester und zu 15,3 % aus 3-Methylheptan-2-on besteht.

### Beispiel 11a

### 2-Methylcapronitril aus 2-(n-Butyl)-cyanessigsäuremethylester - Variation der Reaktionstemperatur

| | |
|---|---|
| 4,65 g | 2-(n-Butyl)-cyanessigsäuremethylester |
| 3,3 g | DMC |
| 0,11 g | Ethyldimethylamin |

wurden auf 125, 150, 175 bzw. 200°C erhitzt und 3 h bei dieser Temperatur gehalten. Der Druck lag bei 10 bis 20 bar. Eine quantitative gaschromatographische Analyse ergab folgende Ausbeuten an Capronitril I, Zwischenprodukt 2-Methyl-2-methoxycarbonylcapronitril II und Verfahrensprodukt 2-Methylcapronitril III (alle Angaben in mol-%).

| Reaktionstemperatur [°C] | I | II | III |
|---|---|---|---|
| 125¹⁾ | 29 | 30 | < 1 % |
| 150 | 29 | 57 | 8 |
| 175 | 26 | 21 | 41 |
| 200 | 16 | 4 | 73 |

| | | | |
|---|---|---|---|
| ¹⁾ 42 % 2-(n-Butyl)-cyanessigsäuremethylester | | | |

### Beispiel 11b

2-Methylcapronitril aus 2-(n-Butyl)-cyanessigsäuremethylester-Variation der Stickstoffbase

4,65 g 2(n-Butyl)-cyanessigsäuremethylester, 3,3 g DMC und 1,5 mmol der in der folgenden Tabelle angegebenen Stickstoffbase wurden unter Rühren 3 h auf 200°C erhitzt. Analog zu Beispiel 11a wurden folgende Ausbeuten bestimmt (Angaben in mol-%).

| Stickstoffbase | I | II | III |
|---|---|---|---|
| 4-Dimethylaminopyridin | 25 | 6 | 60 |
| 1,4-Diazabicyclo[2.2.2]-octan | 20 | 3 | 62 |
| 1,5-Diazabicyclo[4.3.0]-non-5-en | 10 | 1 | 83 |
| Piperidin | 15 | 2 | 75 |
| Di-n-butylamin | 22 | 9 | 61 |
| n-Pentylamin | 28 | 34 | 28 |

### Beispiel 12

### 3-Methylpentan-2-on aus 3-Ethylpentan-2,4-dion

| | |
|---|---|
| 38,4 g | 3-Ethylpentan-2,4-dion |
| 32,4 g | DMC |
| 1,1 g | Ethyldimethylamin |

Es wurden destillativ 41,9 g isoliert, die 4,8 g 3-Methylpentan-2-on sowie 1,53 g Pentan-2-on enthielten.

### Beispiel 13

### 2-Methyl-2-methoxycarbonylcapronitril aus 2-(n-Butyl)-cyanessigsäuremethylester - Isolierung des Zwischenproduktes

| | |
|---|---|
| 46,5 g | 2-(n-Butyl)-cyanessigsäuremethylester |
| 33 g | DMC |
| 1,1 g | Ethyldimethylamin |

Durchführung wie Beispiel 6, aber bei einer Reaktionstemperatur von 150°C und mit einer Reaktionszeit von 3 h.

Der Reaktionsaustrag enthielt (GC-Analyse)

| | |
|---|---|
| 52 % | 2-Methyl-2-methoxycarbonylcapronitril |
| 29 % | Capronitril und |
| 12 % | 2-Methylcapronitril. |

Destillativ wurden 23,7 g 2-Methyl-2-methoxycarbonylcapronitril mit einem Siedepunkt von 58°C/0,8 mbar isoliert (Ausbeute 47 %; bezogen auf die Ausgangsverbindung).

## Patentansprüche

1. Verfahren zur Herstellung von Methylverbindungen der allgemeinen Formel I 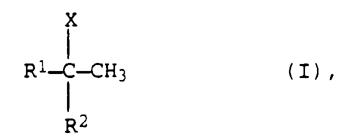 in der die Substituenten folgende Bedeutung haben:
R¹, R² Wasserstoff, unabhängig voneinander oder gemeinsam organischer Rest mit 1 bis 40 Kohlenstoffatomen,
R² 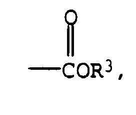 ―CN,
X 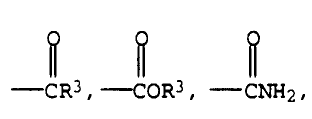 -CN, -SO₂R⁴,
wobei R³ für Wasserstoff oder C₁-C₆-Alkyl steht, R⁴ für C₂-C₆-Alkyl steht oder mit R¹ ein C₃-C₅-Alkylen- oder Alkenylenbrückenglied bildet,
dadurch gekennzeichnet, daß man
a) eine Verbindung der allgemeinen Formel II 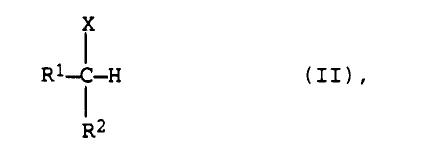 oder
b) für den Fall, daß R² in Verbindung I Wasserstoff bedeutet, eine Verbindung der allgemeinen Formel III 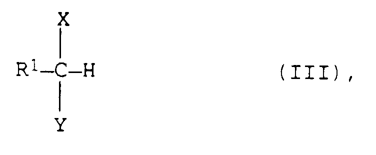 in der Y für 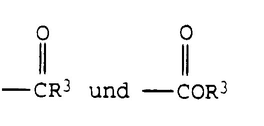 steht,
mit Dimethylcarbonat in Gegenwart einer stickstoffhaltigen Base umsetzt.

2. Verfahren nach Anspruch 1, dadurch gekennzeichnet, daß man als stickstoffhaltige Base Ammoniak, primäre, sekundäre oder tertiäre Amine, die C₁- bis C₃₀-Alkyl-, C₃- bis C₈-Cycloalkyl- oder C₇-C₂₀-Aralkylreste tragen, 1,5-Diazabicyclo-[4.3.0]non-5-en(DBN), 1,8-Diazabicyclo-[5.4.0]undec-7-en (DBU) oder Guanidin verwendet.

3. Verfahren nach den Ansprüchen 1 oder 2, dadurch gekennzeichnet, daß man die Reaktion bei 150 bis 230°C vornimmt.

4. Verfahren nach den Ansprüchen 1 bis 3, dadurch gekennzeichnet, daß man das molare Verhältnis von Dimethylcarbonat zu einer Verbindung der allgemeinen Formel II bzw. III von 2:1 bis 5:1 und das molare Verhältnis einer Verbindung der allgemeinen Formel II bzw. III zu einer stickstoffhaltigen Base von 3:1 bis 20:1 wählt.

## Claims

1. A process for preparing methyl compounds of the general formula I 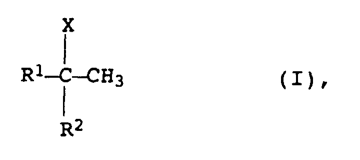 where
R¹ and R² are each hydrogen or independently of one another or together an organic radical having 1 to 40 carbon atoms,
R² is 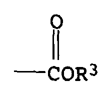 or -CN,
X is 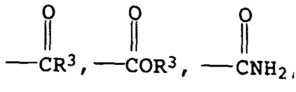 -CN or -SO₂R⁴,
where R³ is hydrogen and C₁-C₆-alkyl, R⁴ is C₂-C₆-alkyl or forms with R¹ a C₃-C₅-alkylene or alkenylene bridge,
which comprises reacting
a) a compound of the general formula II 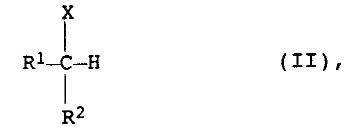 or
b) in the case where R² in compound I is hydrogen, a compound of the general formula III 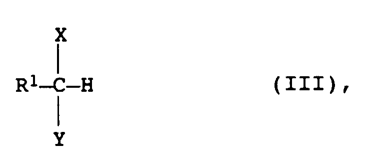 where Y is 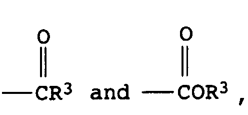
with dimethyl carbonate in the presence of a nitrogen-containing base.

2. A process as claimed in claim 1, wherein ammonia, primary, secondary or tertiary amines which have C₁-C₃₀-alkyl, C₃-C₈-cycloalkyl or C₇-C₂₀-aralkyl radicals, 1,5-diazabicyclo[4.3.0]non-5-ene (DBN), 1,8-diazabicyclo[5.4.0]undec-7-ene (DBU) or guanidine is used as nitrogen-containing base.

3. A process as claimed in claim 1 or 2, wherein the reaction is carried out at from 150 to 230°C.

4. A process as claimed in any of claims 1 to 3, wherein the molar ratio of dimethyl carbonate to a compound of the general formula II or III is from 2:1 to 5:1, and the molar ratio of a compound of the general formula II or III to a nitrogen-containing base is from 3:1 to 20:1.

## Revendications

1. Procédé de préparation de composés méthyliques de la formule générale I 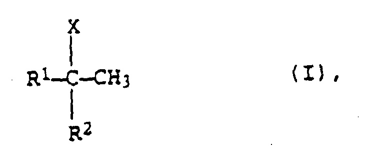 dans laquelle les divers substituants ont les significations qui suivent :
R¹, R² représentent un atome d'hydrogène, indépendamment l'un de l'autre ou en commun, un radical organique comptant de 1 à 40 atomes de carbone,
R² représente un radical 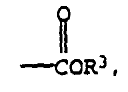 -CN,
X représente un radical 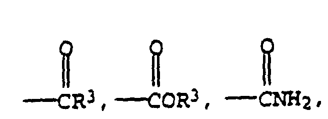 -CN, -SO₂R⁴,
où R³ représente un atome d'hydrogène ou un radical alkyle en C₁ à C₆, R⁴ représente un radical alkyle en C₂ à C₆ ou forme avec R¹ un élément de pontage du type alkylène en C₃ à C₅ ou alcénylène en C₃ à C₅,
caractérisé en ce que
a) on fait réagir un composé de la formule générale II 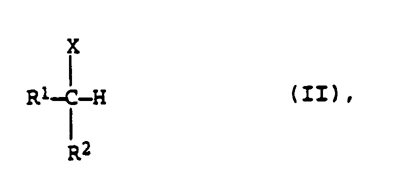 ou
b) dans le cas où R² représente un atome d'hydrogène dans le composé I, un composé de la formule générale III 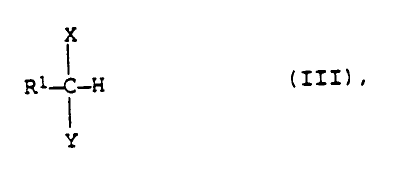 dans laquelle Y représente un radical 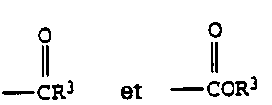
avec le carbonate de diméthyle, en présence d'une base contenant de l'azote.

2. Procédé suivant la revendication 1, caractérisé en ce que l'on utilise, à titre de base contenant de l'azote, de l'ammoniac, des amines primaires, secondaires ou tertiaires, qui portent des radicaux alkyle en C₁ à C₃₀, cycloalkyle en C₃ à C₈, ou aralkyle en C₇ à C₂₀, le 1,5-diazabicyclo-[4.3.0]non-5-ène (DBN), le 1,8-diazabicyclo-[5.4.0]undéc-7-ène (DBU) ou la guanidine.

3. Procédé suivant la revendication 1 ou 2, caractérisé en ce que l'on entreprend la réaction à 150 à 230°C.

4. Procédé suivant les revendications 1 à 3, caractérisé en ce que l'on choisit le rapport molaire du carbonate de diméthyle à un composé de la formule (II) ou (III) de 2:1 à 5:1 et le rapport d'un composé de la formule Il ou III à une base contenant de l'azote de 3:1 à 20:1.
